(19) Europäisches Patentamt
European Patent Office
Office européen des brevets

(11) Veröffentlichungsnummer: **0 510 459 A2**

# (12) EUROPÄISCHE PATENTANMELDUNG

(21) Anmeldenummer: **92106222.0**

(22) Anmeldetag: **10.04.92**

(51) Int. Cl.[5]: **C07C 271/28**, C07C 269/04, C07C 265/14, C07C 263/04

(30) Priorität: **23.04.91 DE 4113156**

(43) Veröffentlichungstag der Anmeldung: **28.10.92 Patentblatt 92/44**

(84) Benannte Vertragsstaaten: **BE DE ES FR GB IT NL**

(71) Anmelder: **BAYER AG**
**W-5090 Leverkusen 1 Bayerwerk(DE)**

(72) Erfinder: **Hammen, Günter, Dr.**
**Bunzlauer Weg 3**
**W-4049 Rommerskirchen 1(DE)**
Erfinder: **Schieb, Thomas, Dr.**
**Vierkotter Feld 1**
**W-5064 Rösrath(DE)**
Erfinder: **Wershofen, Stefan, Dr.**
**Nahestrasse 28**
**W-4050 Mönchengladbach 2(DE)**

## (54) Verfahren zur Herstellung von Poly(O-alkylurethanen) der Di-phenylmethanreihe.

(57) Die Erfindung betrifft ein verbessertes Verfahren zur Herstellung von monomeren und polymeren Poly(O-alkylurethanen) der Diphenylmethanreihe durch Umsetzung der entsprechenden Amine mit Dialkylcarbonaten in Gegenwart eines Katalysators und Isolierung der Produkte mit hoher Reinheit sowie die Verwendung der Produkte zur Herstellung der entsprechenden Isocyanate.

EP 0 510 459 A2

Die vorliegende Erfindung betrifft ein verbessertes Verfahren zur Herstellung von monomeren und polymeren Poly(O-alkylurethanen) der Diphenylmethanreihe durch Umsetzung der entsprechenden Amine mit Dialkylcarbonaten in Gegenwart eines Katalysators und Isolierung der Produkte mit hoher Reinheit sowie die Verwendung der Produkte zur Herstellung der entsprechenden Isocyanate.

Die Herstellung von Urethanen durch Umsetzung von Aminen mit organischen Carbonaten ist bekannt. Zur Aufarbeitung der Reaktionsgemische und Isolierung der Produkte, um einen gewissen Reinheitsgrad zu erhalten, sind in der Literatur verschiedene Verfahrensweisen beschrieben.

Monourethane können z.B. durch Destillation gereinigt und isoliert werden (DE 3 035 354, EP 0 323 514, EP 0 391 473). Diese Methode ist jedoch auf monomere und polymere Poly(O-alkylurethane) der Diphenylmethanreihe nicht anwendbar, da sich diese Urethane nicht unzersetzt destillieren lassen.

Eine andere Möglichkeit zur Reinigung der bei der Umsetzung von Aminen mit Carbonaten gebildeten Urethane ist das Umkristallisieren. Gemäß DE 2 160 111 wird z.B. nach der Reaktion von Anilin mit Dimethylcarbonat als Nebenprodukt entstandener Diphenylharnstoff abfiltriert, das Filtrat eingeengt und der Rückstand aus Hexan umkristallisiert, um N-Phenyl-O-methylurethan zu gewinnen. Das Verfahrensprodukt enthält jedoch auch nach Umkristallisieren als Verunreinigung noch ca. 20 % N-alkylierte Aniline, die als Nebenprodukte bei der Reaktion angefallen sind.

4,4'-Bis(butoxycarbonylamino)diphenylmethan kann gemäß DE 3 035 354 nach der Umsetzung von 4,4'-Diaminodiphenylmethan mit überschüssigem Dibutylcarbonat isoliert werden, indem das Reaktionsgemisch in Dichlormethan aufgenommen wird, Harnstoffe abfiltriert werden, das Filtrat eingeengt und der Rückstand aus einem Toluol-Ligroin-Gemisch umkristallisiert wird. Nachteilig bei diesem Verfahren ist die geringe Ausbeute an Diurethan (17 % d.Th.) und die Bildung großer Mengen an Oligo- und Polyharnstoffen, die in einem zusätzlichen Verfahrensschritt ins Diurethan überführt werden müssen. Zudem ist eine destillative Trennung des beim Einengen anfallenden Gemisches aus Dichlormethan und überschüssigem Dibutylcarbonat erforderlich.

Eine ähnliche Verfahrensweise gibt US 4 550 188 für die Isolierung von 4,4'-Bis-(ethoxycarbonylamino)diphenylmethan an, das bei der Umsetzung von 4,4'-Diaminodiphenylmethan mit überschüssigem Diethylcarbonat gebildet wird. Die Reaktionslösung wird zunächst mit Wasser gemischt, es folgt eine Extraktion mit Dichlormethan. Die organische Phase wird über Natriumsulfat getrocknet und anschließend eingeengt. Nachteilig bei diesem Verfahren ist die aufwendige wäßrige Aufarbeitung unter Verwendung von Dichlormethan als Extraktionsmittel Vor der Rückführung des überschüssigen Diethylcarbonats ist eine destillative Abtrennung vom eingesetzten Extraktionsmittel erforderlich. Zudem enthält das Verfahrensprodukt neben 92,4 % Diurethan als Verunreinigungen noch Aminourethan und Harnstoffe (7,4 %) sowie geringe Mengen an Diamin (0,2 %).

Aufgabe der vorliegenden Erfindung ist daher ein Verfahren zur Herstellung von monomeren und polymeren Poly(O-alkylurethanen) der Diphenylmethanreihe zur Verfügung zu stellen, das die Herstellung und Isolierung der Produkte in hoher Reinheit durch einfache und effiziente Aufarbeitung ermöglicht, ohne daß die oben beschriebenen Nachteile bekannter Verfahren auftreten.

Diese Aufgabe wird mit dem erfindungsgemäßen Verfahren gelöst.

Gegenstand der vorliegenden Erfindung ist ein Verfahren zur Herstellung von monomeren und polymeren Poly(O-alkylurethanen) der Diphenylmethanreihe durch Umsetzung der entsprechenden Amine mit Dialkylcarbonaten in Gegenwart eines Katalysators, welches dadurch gekennzeichnet ist, daß als Amine 4,4'-Diaminodiphenylmethan, 2,4'-Diaminodiphenylmethan, 2,2'-Diaminodiphenylmethan, Homologe der genannten Verbindungen, wie sie z.B. bei der Kondensation von Anilin mit Formaldehyd entstehen, sowie Gemische dieser Verbindungen und als Dialkylcarbonate Carbonate mit $C_1$-$C_2$-Alkylgruppen im Überschuß eingesetzt werden, so daß die gebildeten monomeren und/oder polymeren Poly(O-alkylurethane) beim Abkühlen, gegebenenfalls nach teilweisem Einengen des Reaktionsgemisches, mit hoher Reinheit auskristallisiert und abgetrennt werden.

Für das erfindungsgemäße Verfahren geeignete Amine sind 4,4'-Diaminodiphenylmethan, 2,4'-Diaminodiphenylmethan, 2,2'-Diaminodiphenylmethan, Homologe der genannten Verbindungen, wie sie z.B. bei der Kondensation von Anilin mit Formaldehyd entstehen (im weiteren Text als Polymer-MDA bezeichnet) sowie Gemische dieser Verbindungen.

Weitere Ausgangsmaterialien für das erfindungsgemäße Verfahren sind Dialkylcarbonate der Formel

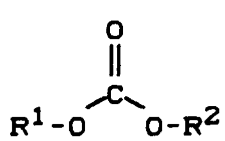

in welcher

$R^1$ und $R^2$ für gleiche oder verschiedene Alkylreste stehen, die 1 bis 2 Kohlenstoffatome aufweisen.

Als Katalysatoren eignen sich Metallverbindungen der 1. bis 5. Hauptgruppe und der 1. bis 8. Nebengruppe des Periodensystems.

Vorzugsweise werden Metallverbindungen der 4. Hauptgruppe und der 2. Nebengruppe des Periodensystems als Katalysatoren eingesetzt.

Besonders bevorzugt werden Verbindungen des Zinns, des Zinks und des Bleis eingesetzt.

Beispiele für derartige Katalysatoren sind

- Lewis-Säuren wie z.B. Salze oder Verbindungen von Zink, Blei, Titan oder Zirkonium
- zinnorganische Verbindungen wie z.B. Dibutylzinnoxid oder Dibutylzinndilaurat
- basische Verbindungen wie z.B. Alkali- oder Erdalkalihydroxide oder -alkoxide.

Bevorzugt sind solche Katalysatoren, die, wenn sie im Produkt verbleiben, eine nachfolgende Spaltung der bei der Umsetzung gebildeten Urethane in die entsprechenden Isocyanate und Alkohole nicht nachteilig beeinflussen.

Die Katalysatoren werden in Mengen von 0,01 bis 20 Mol-%, bevorzugt 0,05 bis 15 Mol-%, besonders bevorzugt 0,1 bis 10 Mol-%, bezogen auf eingesetztes Polyamin, eingesetzt.

Die Reaktionstemperaturen liegen im Bereich von 70 bis 300 ° C, bevorzugt im Bereich von 100 bis 250 ° C. Das Verfahren kann bei Normaldruck oder erhöhtem Druck durchgeführt werden. Erhöhter Druck ist erforderlich, wenn niedrigsiedende Reaktionspartner bei über ihrem Siedepunkt liegenden Temperaturen umgesetzt werden sollen.

Bei der Umsetzung eines Polyamins mit einem Dialkylcarbonat kommen die Reaktionspartner im allgemeinen in solchen Mengen zum Einsatz, daß auf jedes Grammäquivalent an Aminogruppen des Polyamins 1 Mol an Dialkylcarbonat entfällt, Bei der Durchführung des erfindungsgemäßen Verfahrens werden die Dialkylcarbonate im Überschuß eingesetzt, da überschüssiges Dialkylcarbonat u.a. als Lösungsmittel dient. Der sich während der Umsetzung gemäß folgender Reaktionsgleichung

$$R\text{-}(NH_2)_n + n(R'O)_2CO \rightarrow R\text{-}(NHCOOR')_n + nR'OH$$

(die Reste R und R' stehen in dieser lediglich das Reaktionsprinzip erläuternden Gleichung für die indifferenten Reste der Reaktionspartner) bildende Alkohol wird entweder fortlaufend während der Reaktion oder zu einem geeigneten Zeitpunkt nach beendeter Umsetzung destillativ abgetrennt. Verbleibt der Alkohol während der Umsetzung im Reaktionsgemisch, werden intermediär gebildete Harnstoffe durch den Alkohol ins Urethan überführt, so daß auf eine Isolierung und separate Umsetzung der Harnstoffe im allgemeinen verzichtet werden kann.

Überraschend wurde gefunden, daß bei Verwendung eines in Abhängigkeit von den Reaktionspartnern geeigneten Dialkylcarbonat-Überschussesdie beim erfindungsgemäßen Verfahren gebildeten monomeren und/oder polymeren Poly(O-alkylurethane) beim Abkühlen, gegebenenfalls nach teilweisem Einengen des Reaktionsgemisches kristallisiert und ohne weitere Reinigung mit hoher Reinheit isoliert werden können. Die isolierten Verfahrensprodukte können einen Teil des eingesetzten Katalysators enthalten. Die nachfolgende thermische Spaltung der Urethane zu den entsprechenden Isocyanaten wird dadurch nicht nachteilig beeinflußt.

Das nach dem Abtrennen der Verfahrensprodukte verbleibende Filtrat enthält neben einem geringen Anteil an monomeren und/oder polymeren Poly(O-alkylurethanen) im wesentlichen Aminourethane und andere Neben- und Zwischenprodukte, die sich durch erneute Umsetzung mit Dialkylcarbonaten in monomere und/oder polymere Poly(O-alkylurethane) überführen lassen, sowie gegebenenfalls geringe Mengen an N-alkylierten Verbindungen. Das Filtrat kann, gegebenenfalls nach Abtrennung von bei der Umsetzung gebildetem Alkohol und von einem Teil der N-alkylierten Nebenprodukte, erneut dem erfindungsgemäßen Verfahren zugeführt werden.

Das erfindungsgemäße Verfahren kann kontinuierlich oder diskontinuierlich durchgeführt werden.

Die nach dem erfindungsgemäßen Verfahren hergestellten Urethane können ohne weitere Aufarbeitung zur Herstellung der entsprechenden Isocyanate verwendet werden.

Dabei werden die Urethane durch thermische Spaltung in die entsprechenden Isocyanate und Alkohole überführt und die entstehenden Spaltprodukte nachfolgend getrennt.

Das erfindungsgemäße Verfahren soll anhand der nachfolgenden Beispiele näher erläutert werden.

## Beispiel 1

Ein Gemisch von 79,3 g (0,4 Mol) 4,4'-Diaminodiphenylmethan, 1816 g (20,0 Mol) Dimethylcarbonat und 1,73 g (2,0 mmol) Octa-Soligen Pb 24 wird in einem 3 l Autoklaven auf 200 ° C aufgeheizt. Nach 30 min. wird die Reaktion durch Abkühlen abgebrochen (Ausbeute: 94 %, Selektivität: 98 %). Anschließend wird langsam auf 5 ° C abgekühlt und der ausgefallene Feststoff filtriert. Nach Auswaschen mit 100 ml Dimethylcarbonat und Trocknen erhält man 99,3 g 4,4'-Bis(methoxycarbonylamino)-diphenylmethan (Reinheit 99 %, Schmelzpunkt 185 ° C), entsprechend einer Ausbeute von 79 % der Theorie. Durch Rückführung des Filtrats kann die Ausbeute an isoliertem 4,4'-Bis-(methoxycarbonylamino)diphenylmethan auf über

95 % der Theorie gesteigert werden.

Beispiel 2

Ein Gemisch von 79,3 g (0,4 Mol) 4,4'-Diaminodiphenylmethan, 1890 g (16,0 Mol) Diethylcarbonat und 3,46 g (4,0 mmol) Octa-Soligen Pb 24 wird in einem 3 l Autoklaven auf 200 °C aufgeheizt. Nach 2 Stunden wird die Reaktion durch Abkühlen abgebrochen (Ausbeute: 89 %, Selektivität: 98 %). Das Reaktionsgemisch wird nach Filtration im Wasserstrahlvakuum eingeengt. Der ausgefallene Feststoff wird abfiltriert, mit 100 ml Diethylcarbonat gewaschen und getrocknet. Man erhält 108,6 g 4,4'-Bis-(ethoxycarbonylamino)diphenylmethan (Reinheit 99 %, Schmelzpunkt 131 °C), entsprechend einer Ausbeute von 79 % der Theorie. Durch Rückführung der Filtrate kann die Ausbeute an isoliertem 4,4'-Bis(ethoxycarbonylamino)diphenylmethan auf über 95 % der Theorie gesteigert werden.

**Patentansprüche**

1. Verfahren zur Herstellung von reinen monomeren und/oder polymeren Poly(O-alkylurethanen) der Diphenylmethanreihe durch Umsetzung von polyfunktionellen Aminen mit Dialkylcarbonaten in Gegenwart eines Katalysators, dadurch gekennzeichnet, daß als polyfunktionelle Amine 4,4'-Diaminodiphenylmethan, 2,4'-Diaminodiphenylmethan, 2,2'-Diaminodiphenylmethan, Homologe der genannten Verbindungen, wie sie z.B. bei der Kondensation von Anilin mit Formaldehyd entstehen, sowie Gemische dieser Verbindungen und als Dialkylcarbonate Carbonate mit $C_1$-$C_2$-Alkylgruppen im Überschuß eingesetzt werden, so daß die gebildeten monomeren und/oder polymeren Poly(O-alkylurethane) durch Abkühlen, gegebenenfalls nach teilweisem Einengen des Reaktionsgemischs, mit hoher Reinheit kristallisiert und abgetrennt werden.

2. Verfahren gemäß Anspruch 1, dadurch gekennzeichnet, daß als Katalysatoren Metallverbindungen der 1. bis 5. Hauptgruppe und der 1. bis 8. Nebengruppe des Periodensystems eingesetzt werden.

3. Verfahren gemäß einem der Ansprüche 1 bis 2, dadurch gekennzeichnet, daß als Katalysatoren Metallverbindungen der 4. Hauptgruppe und der 2. Nebengruppe des Periodensystems eingesetzt werden.

4. Verfahren gemäß einem der Ansprüche 1 bis 3, dadurch gekennzeichnet, daß als Katalysatoren Verbindungen des Zinns, des Zinks und des Bleis eingesetzt werden.

5. Verwendung der nach dem Verfahren gemäß einem der Ansprüche 1 bis 4 hergestellten Urethane ohne weitere Aufarbeitung zur Herstellung der entsprechenden Isocyanate durch Spaltung.